# EUROPÄISCHE PATENTANMELDUNG

(11) **EP 1 077 208 A2**
(43) Veröffentlichungstag der Anmeldung: **21.02.2001**
(21) Anmeldenummer: 00116203.1
(22) Anmeldetag: 03.08.2000
(51) Int. Cl.: C07C 69/65, C07C 51/38, C07C 67/343, C07C 67/32, C07C 67/54, C07C 67/56, C07C 57/58

(54) **Verfahren zur Herstellung von (Bis-(trifluormethyl)-phenyl)-essigsäuren und deren Alkylestern sowie (Bis-(trifluormethyl)-phenyl)-malonsäure-dialkylester**

(30) Priorität: 16.08.1999 DE 19938736
(71) Anmelder: BAYER AG, 51368 Leverkusen (DE)
(72) Erfinder: Marhold, Albrecht, Dr., 51373 Leverkusen (DE); Stölting, Jörn, Dr., 51061 Köln (DE)

(57) **Zusammenfassung**

[Bis-(trifluormethyl)-phenyl]-essigsäuren werden in vorteilhafter Weise erhalten, wenn man ein entsprechendes Brom- oder Iod-bis-(trifluormethyl)-benzol mit einem Malonsäure-di-C₁-C₄-alkylester in Gegenwart eines Deprotonierungsagenzes und eines Kupfersalzes umsetzt und das Reaktionsprodukt in basischem Medium verseift und decarboxyliert. Ein Gemisch von [Bis-(trifluormethyl)-phenyl-essigsäurealkylester und [Bis-(trifluormethyl)-phenyl]-malonsäurealkylester kann erhalten werden, wenn das vor der Verseifung und vollständigem Decarboxylierung vorliegende Reaktionsgemisch mit Wasser und Säure versetzt und erwärmt. Aus dem Gemisch kann man [Bis-(trifluormethyl)-phenyl]-essigsäurealkylester erhalten, indem man es bei vermindertem Druck destilliert und [Bis-(trifluormethyl)-phenyl]-malonsäuredialkylester, wenn man es mittels Säulenchromatographie, fraktionierte Destillation oder Dünnschichtdestillation aufarbeitet.

[Bis-(trifluormethyl)-phenyl]-malonsäureester sind neue Verbindungen.

## Beschreibung

Die vorliegende Erfindung betrifft ein Verfahren zur Herstellung von [Bis-(trifluormethyl)-phenyl]-essigsäuren und deren Alkylestern aus Brom-bis-(trifluormethyl)benzolen und Malonsäure-dialkylestern und dabei als Zwischenprodukt erhältliche [Bis-(trifluormethyl)-phenyl]-malonsäure-dialkylester.

Bis-(trifluormethyl)-substituierte Phenylessigsäuren und deren Derivate haben Bedeutung als Zwischenprodukte für pharmazeutische Wirkstoffe (siehe z.B. US 5,817,658, WO 98/00405, WO 98/07722, WO 95/21819 und US 5,696,267).

In WO 96/05827 wird die Herstellung der 2,4-Bis-(trifluormethyl)-phenylessigsäure durch saure Hydrolyse des entsprechenden Phenylacetonitrils beschrieben. Die Ausbeute ist mit 68,5 % wenig zufriedenstellend. Das Nitril wurde aus dem nur schwierig zugänglichen 2,4-Bis(-trifluormethyl)-benzylbromid durch Umsetzung mit einem großen Überschuß an Natriumcyanid hergestellt. Die Toxizität des Natriumcyanids erfordert erheblichen arbeitshygienischen Aufwand bei seiner Handhabung und Entsorgung.

3,5-Bis(trifluormethyl)-phenylessigsäuremethylester kann durch Veresterung der entsprechenden Säure mit Thionylchlorid in Gegenwart von Methanol hergestellt werden (WO 95/21819). Zum Aufbau der Bis-(trifluormethyl)-phenylessigsäure-Struktur wird dabei nichts gesagt.

Der gleiche Ester kann auch durch basische Verseifung und saure Aufarbeitung in rund 75 % der Theorie aus 1-β,β,β-Trichlorethyl-3,5-bis-(trifluormethyl)-benzol erhalten werden. Dieses wiederum wurde durch Umsetzung von in Salzsäure diazotiertem 3,5-Bis-(trifluormethyl)-anilin mit Vinylidenchlorid mit 73 % Ausbeute erhalten (DE 33 14 249). Es ist allgemein bekannt, daß die Ausbeuten bei dieser Art von Reaktion nicht reproduzierbar sind und großen Schwankungen unterliegen. Bei der Nacharbeitung dieses Verfahrens wurden nur Ausbeuten von knapp 10 bis knapp 50 % der Theorie erreicht. Darüber hinaus ist Vinylidenchlorid (1,1-Dichlorethen) ein krebserzeugender Stoff, der nur mit erheblichem Aufwand gehandhabt werden kann.

Es besteht deshalb nach wie vor ein Bedürfnis nach einem einfachen Verfahren zur Herstellung von [Bis-(trifluormethyl)-phenyl]-essigsäuren und deren Alkylestern in hohen Ausbeuten, bei dem weniger toxische und keine krebserzeugenden Stoffe gehandhabt werden müssen und von gut zugänglichen Ausgangsprodukten ausgegangen wird.

Es wurde nun ein Verfahren zur Herstellung von [Bis-(trifluormethyl)-phenyl]-essigsäuren gefunden, das dadurch gekennzeichnet ist, daß man ein entsprechendes Brom- oder Iod-bis-(trifluormethyl)-benzol mit einem Malonsäure-di-C₁-C₄-alkylester in Gegenwart eines Deprotonierungsagenzes und eines Kupfersalzes umsetzt und das Reaktionsprodukt in basischem Medium verseift und decarboxyliert.

In das erfindungsgemäße Verfahren kann man beispielsweise Brom- oder Iod-bis(trifluormethyl)-benzole der Formel (I) einsetzen 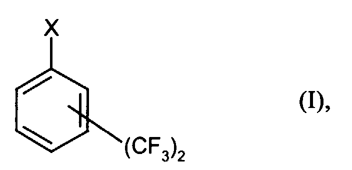
in der X für Brom oder Iod steht,
und [Bis-(trifluormethyl)-phenyl]-essigsäuren der Formel (II) herstellen

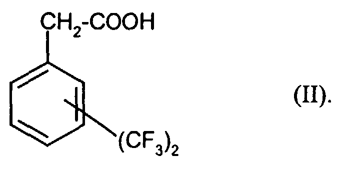

Vorzugsweise werden Brom-bis(trifluormethyl)-benzole der Formel (I) eingesetzt.

Als Malonsäure-di-C₁-C₄-alkylester kommen solche der Formel (III) infrage

ROOC-CH₂-COOR (III),

in der die beiden Reste R gleich oder verschieden sind und jeweils für C₁-C₄-Alkyl stehen. Vorzugsweise sind die beiden Reste R gleich und stehen für Methyl, Ethyl, n-Propyl, i-Propyl und n-Butyl.

In den Formeln (I) und (II) befinden sich die beiden Trifluormethylgruppen vorzugsweise in 2,4- oder 3,5-Stellung zum Bromatom bzw. zur CH₂-COOH-Gruppe. Besonders bevorzugt befinden sie sich in 3,5-Stellung.

Die zur Durchführung des erfindungsgemäßen Verfahrens benötigten Brom-bis-(trifluormethyl)-benzole und Malonsäure-di-C₁-C₄-alkylester sind bekannt und kommerziell erhältlich oder nach bekannten Verfahren oder analog dazu herstellbar.

Für das erfindungsgemäße Verfahren kommen als Deprotonierungsagenz anorganische und organische Basen infrage, beispielsweise Erdalkali- und Alkalimetallhydride, -amide, -alkoholate, -carbonate und -hydrogencarbonate wie Natriumhydrid, Natriumamid, Natrium-methylat, Natrium-ethylat, Kalium-tert.-butylat, Natrium-, Kalium- und Ammoniumcarbonat, Natrium- und Kaliumhydrogencarbonat sowie tertiäre Amine wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, 1,4-Diazabicyclo[2.2.2]octan (DABCO), 1,5-Diazabicyclo[5.4.0]non-5-en (DBN) und 1,8-Diazabicyclo[5.4.0]undec-7-en (DBU). Man kann auch Kombinationen verschiedener Basen einsetzen. Bevorzugt setzt man Alkalialkoholate ein, besonders bevorzugt das Alkoholat des Alkohols R-OH, bei dem R die gleiche Bedeutung hat wie im eingesetzten Malonsäure-di-C₁-C₄-alkylester. Alkoholate kann man z.B. in fester Form oder gelöst in einem Alkohol, vorzugsweise in dem Alkohol, der dem Alkoholatanion entspricht, einsetzen.

Als Kupfersalze kommen z.B. Kupfer(I)-salze infrage wie Kupfer(I)-halogenide. Vorzugsweise setzt man Kupfer(I)-bromid oder Kupfer(I)-iodid oder eine Mischung beider Salze ein.

Das erfindungsgemäße Verfahren kann in Gegenwart eines Verdünnungsmittels durchgeführt werden. Hierfür kommen organische Lösungsmittel in Betracht, die das erfindungsgemäße Verfahren nicht negativ beeinflussen und beliebige Mischungen davon. Beispielhaft seien genannt: aromatische Kohlenwasserstoffe wie Toluol, Xylol und Mesitylen, halogenierte aromatische Kohlenwasserstoffe wie Chlorbenzol und Dichlorbenzole, Ether wie Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether und Anisol, Amide wie N,N-Dimethylformamid, N,N-Dimethylacetamid, N-Methylformanilid, N-Methylpyrrolidin und Hexamethylphosphorsäuretriamid, N-Oxide wie N-Methyl-morpholin-N-oxid, Ester wie Methyl-, Ethyl- und Butylacetat, Sulfone wie Sulfolan und Alkohole wie Methanol, Ethanol, n- und i-Propanol, n-, i-, s- und t-Butanol, Ethandiol, Propan-1,2-diol, Ethoxyethanol, Methoxyethanol, Diethylenglykolmonomethylether und Diethylenglykolmonoethylether.

Pro Mol Brom-bis-(trifluormethyl)-benzol kann man beispielsweise 1,0 bis 2,5 Mol, vorzugsweise 1,1 bis 1,6 Mol Malonsäure-di-C₁-C₄-alkylester und 0,1 bis 1 Mol, vorzugsweise 0,3 bis 0,5 Mol Kupfersalze sowie gegebenenfalls 0,1 bis 2 l Verdünnungsmittel verwenden. Das Deprotonierungsagenz kann man beispielsweise in einer Menge von 85 bis 110 % der dem eingesetzten Malonester äquivalenten Menge einsetzen. Vorzugsweise liegt diese Menge bei 90 bis 99 %.

Das erfindungsgemäße Verfahren kann man beispielsweise bei 40 bis 150°C durchführen. Bevorzugt arbeitet man bei 50 bis 110°C.

Bei einer bevorzugten Ausführungsform des erfindungsgemäßen Verfahrens arbeitet man wie folgt: Man legt entweder den Malonsäureester oder das Deprotonierungsagenz, gegebenenfalls in einem der angegebenen Verdünnungsmittel vor, erwärmt auf 40 bis 70°C und dosiert die jeweils andere dieser beiden Komponenten zu. Anschließend kann man, beispielsweise beim Einsatz von Alkoholaten als Deprotonierungsagenzien, andestillieren um den entstandenen Alkohol teilweise zu entfernen und danach mindestens auf einige Grad unter den Siedepunkt des Destillationssumpfes abkühlen. Man gibt Kupfersalz und das Brom-bis-(trifluormethyl)-benzol zu und bringt das Gemisch auf die gewünschte Reaktionstemperatur. Wenn man ein Verdünnungmittel eingesetzt hat, entspricht die Reaktionstemperatur vorzugsweise der Rückflußtemperatur des Verdünnungsmittels. Während der Reaktion ist Gasentwicklung zu beobachten, die durch eine partielle Demethoxycarbonylierung hervorgerufen wird. Man kann auch nach der Zugabe des Kupfersalzes die Reaktionstemperatur einstellen und dann das Brom-bis-(trifluormethyl)-benzol zudosieren, was bei stärkerer Gasentwicklung zu bevorzugen ist. Die Reaktion ist, im wesentlichen abhängig von der Temperatur, i.a. nach 1 bis 50 Stunden beendet.

Man kann auch einen Teil des Kupfersalzes, beispielsweise 5 bis 30 % davon, vorher abzweigen und erst nach Ablauf eines Teils der Reaktionszeit zusetzen. Durch eine derartige Zugabe von frischem Katalysator während der Reaktion kann i.a. die Gesamtreaktionszeit reduziert werden.

Die Verseifung und vollständige Decarboxylierung kann z.B. auf folgende Weise durchgeführt werden: Zur Herstellung von [Bis-(trifluormethyl)-phenyl]-essigsäuren ohne Isolierung der Zwischenprodukte kann man nach der Reaktion und der Entfernung der Kupfersalze z.B. durch Filtration direkt mit wäßrigem Alkali, beispielsweise mit 30 bis 50 gew.-%iger wäßriger Natronlauge, versetzen und erwärmen, z.B. auf eine Temperatur von höchstens 120°C. Die Isolierung des Produkts kann dann nach üblichen Methoden durchgeführt werden, beispielsweise zunächst durch Einengen, vorzugsweise unter Vakuum, Ansäuern des Rückstands mit eine Mineralsäure wie Salzsäure und Abtrennung des Produkts durch Filtrieren oder Zentrifugieren. Man kann so [Bis-(trifluormethyl)-phenyl]-essigsäuren in Ausbeuten von beispielsweise 85 % der Theorie und mehr erhalten.

Zur Herstellung von [Bis-(trifluormethyl)-phenyl]-essigsäureestern ohne Isolierung der Zwischenprodukte kann man nach der Reaktion und vor der Entfernung der Kupfersalze auch anders verfahren. So kann man das vorliegende Gemisch abkühlen, z.B. auf eine Temperatur, die nicht niedriger als 10°C ist, und dann mit Säure neutralisieren, d.h. z.B. einen pH-Wert im Bereich von 5 bis 9 einstellen. Als Säuren kommen Mineral- und Carbonsäuren infrage, bevorzugt ist Essigsäure. Nunmehr kann man die festen Bestandteile des Reaktionsgemisches abtrennen, z.B. durch Filtration, vorzugsweise unter Verwendung eines Filterhilfsmittels, und den Rückstand mit einem Ether oder Alkohol waschen. Vorzugsweise nimmt man hierfür Dioxan. Aus dem mit der Waschflüssigkeit vereinten Filtrat kann man dann das Produkt durch Destillation unter vermindertem Druck gewinnen. Die Destillation führt man vorzugsweise nach Zusatz einer hochsiedenen, inerten Flüssigkeit durch, um vor allem gegen Ende der Destillation den Wärmeübergang zu verbesseren. Diese Aufarbeitungsmethode ist die bevorzugte, auch zur Herstellung von (Bis-[trifluormethyl-phenyl]-essigsäuren, die man aus dem so isolierten entsprechenden Estern durch eine übliche Esterverseifung erhalten kann.

Bei der erfindungsgemäßen Herstellung von [Bis-(trifluormethyl)-phenyl]-essigsäuren werden als Zwischenstufen die entsprechenden Phenylmalonsäurendialkylester und die entsprechenden Phenylessigsäurealkylester durchlaufen. Ein Gemisch beider kann man isolieren, wenn man das vor der Verseifung und vollständigen Decarboxylierung vorliegende Reaktionsgemisch mit Wasser und Säure versetzt und erwärmt, z.B. auf eine Temperatur von höchstens 130°C. Als Säuren kommen z.B. Mineral- und Carbonsäuren infrage. Nach einer üblichen Aufarbeitung, z.B. Abkühlen, mit Wasser verdünnen, mit organischem Lösungsmittel extrahieren und destillieren des Extrakts nach Abtrennung des Extraktionsmittels, kann man ein Gemisch erhalten, das den entsprechenden Phenylmalonsäuredialkylester der Formel (IV) und den entsprechenden Phenylessigsäurealkylester der Formel (V) enthält und praktisch frei ist von der entsprechenden freien Säure der Formel (II).

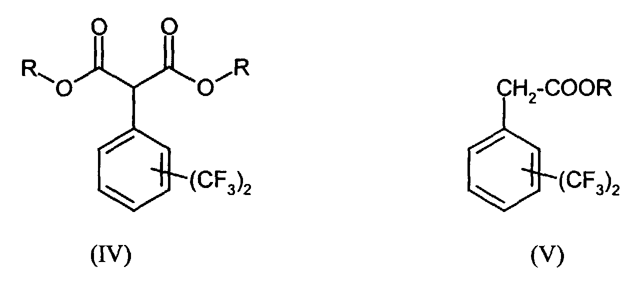

In den Formeln (IV) und (V) haben die Reste R die gleiche Bedeutung wie bei Formel (III) angegeben.

In dem isolierten Estergemisch ist mehr Phenylmalonsäuredialkylester der Formel (IV) enthalten, wenn man die Behandlung mit Wasser und Säure nur kurz, z.B. für 1 bis 20 Minuten, und bei relativ tiefen Temperaturen, z.B. 10 bis 30°C, durchführt. Entsprechend enthält das isolierte Estergemisch mehr Phenylessigsäuredialkylester der Formel (V), wenn man die Behandlung mit Wasser und Mineralsäure länger, z.B. für 30 Minuten bis 5 Stunden, und bei relativ höheren Temperaturen, z.B. 30 bis 130°C, durchführt.

Aus dem isolierten Estergemisch läßt sich der jeweilige Phenylmalonsäuredialkylester der Formel (IV) beispielsweise gewinnen, indem man mittels Säulenchromatographie, fraktionierter Destillation oder Dünnschichtdestillation aufarbeitet. Bei destillativen Methoden reichert sich der Phenylmalonsäuredialkylester der Formel (IV) im Sumpf an.

Aus dem isolierten Estergemisch läßt sich der jeweilige Phenylessigsäurealkylester der Formel (V) beispielsweise durch Destillation bei vermindertem Druck gewinnen, z.B. siedet der [3,5-Bis-(trifluormethyl)-phenyl]-essigsäuremethylester bei 9 mbar bei 89 bis 99°C.

Aus dem isolierten Estergemisch kann man z.B. durch Zugabe eines protischen Lösungsmittels, z.B. Wasser oder Alkohol wie Methanol, Alkali, z.B. 10 bis 30 gew.-%ige wäßrige Natronlauge und Erwärmung auf z.B. 30 bis 80°C und Ansäuern mit einer Mineralsäure die entsprechende Phenylessigsäure der Formel (II) erhalten. Wenn diese das gewünschte Produkt ist, kann die Zwischenisolierung des Estergemisches natürlich unterbleiben, da sie unnötigen Aufwand und eine Ausbeuteminderung zur Folge hat.

Die Phenylmalonsäuredialkylester der Formel (IV) sind neu. Die vorliegende Erfindung betrifft deshalb auch [Bis-(trifluormethyl)-phenyl]-malonsäuredialkylester der Formel (IV) 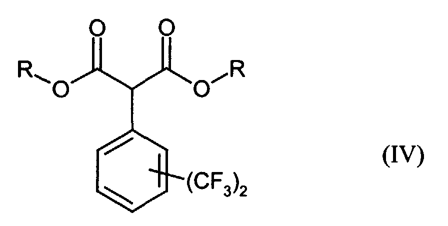 in der die beiden Reste R gleich oder verschieden sind und jeweils für C₁-C₄-Alkyl stehen. Vorzugsweise sind die beiden Reste R gleich und stehen für Methyl, Ethyl, n-Propyl, i-Propyl oder n-Butyl.

Diese Malonester ermöglichen, wenn sie anstelle der üblichen Malonester eingesetzt werden, Möglichkeiten zur Herstellung von bekannten Wirkstoffen durch neue Verfahren und von neuen Wirkstoffen. Sie bereichern dadurch die Technik. Kernsubstituierte Arylmalonester eignen sich insbesondere zum Aufbau von Triazolopyrimidinen, die Breitspektrumsfungizide im Pflanzenschutz darstellen (siehe EP-A 83 451).

### Beispiele

### Beispiel 1

### [3,5-Bis-(trifluormethyl)-phenyl]-essigsäure

550 ml Ethylenglykoldimethylether wurden vorgelegt und unter Stickstoff 91 g Natriummethylat (fest) eingetragen, wobei ohne Temperaturänderung eine gut ruhrbare weiße Suspension entstand. Die Mischung wurde auf 69°C erwärmt und 237,6 g Malonsäuredimethylester innerhalb von 30 Minuten zugetropft., wobei das Gemisch am Rückfluß zu Sieden begann (74°C Innentemperatur) und ein rührbarer, weißer Brei entstand. Nach Abkühlung auf 54°C wurden 35,2 g Kupfer(I)-bromid (feines Pulver) und 35,2 g Kupfer(I)-iodid (feines Pulver) zugegeben und anschließend erneut zum Sieden am Rückfluß erhitzt. Innerhalb von 110 Minuten wurden 344 g 3,5-Bis-(trifluormethyl)-brombenzol zugetropft, wobei ein leichter Abgasstrom beobachtet wurde (partielle Demethoxycarbonylierung). Nach 15 Stunden und 40 Minuten (GC-Umsatzkontrolle) bei Rückfluß (85°C) wurden weitere 10 g Kupfer(I)-bromid und weitere 10 g Kupfer(I)-iodid zugegeben. Nach weiteren 5 Stunden am Rückfluß bei 86°C (GC-Umsatzkontrolle; mindestens 90 % Umsatz des eingesetzten Brombenzols) wurden 400 g 45 gew.-%ige wäßrige Natriumhydroxidlösung bei 86°C (Rückfluß) innerhalb von 15 Minuten zugetropft und weitere 4 Stunden bei dieser Temperatur unter Rückfluß gehalten, wobei eine leichte Abgasentwicklung stattfand. Danach zeigte eine GC-Analyse, daß kein Ester mehr vorlag. Es wurden unter Rühren 1 000 ml Wasser zugesetzt und der Niederschlag absitzen gelassen. Danach dekantierte man vom Ungelöstem über eine Nutsche weitgehendst ab, schlämmte den zurückgebliebenden Kolbeninhalt mit 100 ml 2 N Natronlauge auf, und saugte über die Nutsche ab. Das so erhaltene zweiphasige Filtrat wurde bei 20 mbar eingedampft (1 350 ml Destillat) und mit 1 000 ml Wasser rückverdünnt. Man extrahierte organische Verunreinigungen mit 30 ml Toluol (1,4 g Rückstand beim Einrotieren) und goß die wäßrige Phase in 920 g 24 gew.-%iger wäßriger Schwefelsäure ein (18 - 24°C), (starkes Aufschäumen, pH-Wert = 1). Der gebildete Niederschlag wurde abgesaugt, mit wenig Wasser neutral gewaschen und bei 60°C über Nacht getrocknet. Es wurden 284 g beiges Pulver erhalten, was einer Ausbeute von 89 % der Theorie entspricht.

### Beispiel 2

### [3,5-Bis-(trifluormethyl)-phenyl]-essigsäuremethylester und -malonsäuredimethylester

2 kg Malonsäuredimethylester wurden in 6 l Dioxan bei 50°C vorgelegt. Dazu wurden in einer Stunde 700 g Natriummethylat portionsweise eingetragen, wobei der Ansatz zunächst breiig, dann wieder leicht rührbar wurde. Nach Ende der Zugabe wurde 1 Stunde bei 50 bis 55°C nachgerührt und anschließend andestilliert bis am Kopf der Siedepunkt von Dioxan (101°C) erreicht wurde (ca. 11 Destillat). Der Ansatz wurde auf 90°C abgekühlt, je 352 g Kupfer(I)-bromid und Kupfer(I)-iodid sowie 3 440 g 3,5-Bis-(trifluormethyl)-brombenzol zugegeben und anschließend 15 Stunden unter Rückfluß reagieren gelassen, wobei eine deutliche Gasentwicklung aufttrat. Dann wurden bei Rückflußbedingungen 2 929 ml Wasser und 2 344 ml konz. Salzsäure zulaufen gelassen und noch 1 Stunde am Rückfluß gekocht. Der Ansatz wurde hiernach auf Raumtemperatur abgekühlt, mit 5 l Wasser verdünnt und mit tert.-Butylmethylether extrahiert (2 x mit je 3 l). Die organische Phase wurde zweimal mit 3 l Wasser gewaschen, getrocknet und destilliert. Es wurden 2 475 g bei 91 bis 125°C und 18 mbar übergehendes Produkt erhalten. Das Produkt bestand zu 53 % (GC, Flächen-%) aus [3,5-Bis-(trifluormethyl)-phenyl]-malonsäuredimethylester (Siedepunkt bei 10 mbar: 128°C) und zu 47 % (GC, Flächen-%) aus [3,5-Bis-(trifluormethyl)-phenyl]-essigsäuremethylester (Siedepunkt bei 9 mbar 89 bis 99°C).

126,2 mg dieses Produktes wurden in 4 ml Acetonitril gelöst und 24 Injektionen zu je 125 µl auf ein RP-18 Chromatographiesäule aufgegeben (das entspricht 94,7 mg des Produkts) und getrennt. Nach Extraktion mit Diethylether wurden aus den vereinigten Fraktionen 7,0 mg [3,5-Bis-(trifluormethyl)-phenyl]-malonsäuredimethylester mit folgenden charakteristischen Merkmalen erhalten:
IR-Spektrum (Film, Wellenzahlen in cm⁻¹):
   1744, 1379, 1280, 1175, 1135, 683.
Massenspektrum (GC/MS):
   344 (M⁺), 325, 300, 257, 227, 59.
¹H-NMR-Spektrum (chemische Verschiebung in ppm):
   3.80 (6H), 4.77 (1H), 7.88 (1H), 7.90 (2H).
¹⁹F-NMR-Spektrum (chemische Verschiebung in ppm):
   -63.35.

### Beispiel 3

### [3,5-Bis-(trifluormethyl)-phenyl]-essigsäure

1 kg des Estergemisches, das gemäß Beispiel 2 erhalten worden war, wurde in 1,5 l Methanol vorgelegt und bei 10°C tropfenweise mit 2 kg 20 gew.-%iger wäßriger Natronlauge versetzt, wobei die Temperatur auf ca. 60°C anstieg. Anschließend wurde 4 Stunden bei 60°C nachgerührt. Der Ansatz wurde im Vakuum eingeengt und in der Kälte mit Salzsäure angesäuert, wobei die Carbonsäure ausfiel. Es wurde abgesaugt, mit Wasser gewaschen und getrocknet. Man erhielt 844 g (65 % der Theorie über beide Stufen) [3,5-Bis-(trifluormethyl)-phenyl]-essigsäure mit einem Schmelzpunkt von 126 bis 128°C.

### Beispiel 4

### [3,5-Bis-(trifluormethyl)-phenyl]-essigsäuremethylester

In eine Mischung aus 550 ml Dioxan und 287,6 g Malonsäuredimethylester wurden bei 69°C 306 g 30 gew.-%ige Natriummethylatlösung in Methanol innerhalb von 30 Minuten zugetropft. Die Mischung begann dabei am Rückfluß zu sieden und es entstand ein rührbarer weißer Brei. Danach wurde auf 54°C abgekühlt und als feine Pulver je 25,2 g Kupfer(I)-bromid und Kupfer(I)-iodid zugegeben und wieder zum Sieden am Rückfluß erhitzt. Innerhalb von 110 Minuten wurden 344 g 3,5-Bis-(trifluormethyl)-brombenzol zugetropft und 10 Stunden bei unveränderter Temperatur nachgerührt. Dann wurden je 10 g Kupfer(I)-bromid und -iodid nachdosiert, 5 Stunden bei 86°C gehalten, nochmals die gleichen Mengen Kupfer(I)-bromid und -iodid nachdosiert und nach 5 Stunden nachrühren unter Rückfluß auf Raumtemperatur abgekühlt. Mit Eisessig wurde danach auf einen pH-Wert von 7 eingestellt, das Gemisch über Celite® filtriert, der Rückstand mit Dioxan gewaschen und das Filtrat mit der Waschflüssigkeit vereinigt und nach Zusatz eines hochsiedenden Weißöles bei 18 mbar über eine Vigreux-Kolonne mit aufgesetzter Brücke destilliert. Bei 18 mbar und 91 bis 125°C wurde das Produkt in einer Ausbeute von 52 % d.Th. erhalten.

Durch eine übliche Esterverseifung kann man aus dem isolierten Ester die entsprechende Säure erhalten.

## Patentansprüche

1. Verfahren zur Herstellung von [Bis-(trifluormethyl)-phenyl]-essigsäuren, dadurch gekennzeichnet, daß man ein entsprechendes Brom- oder Iod-bis-(trifluormethyl)-benzol mit einem Malonsäure-di-C₁-C₄-alkylester in Gegenwart eines Deprotonierungsagenzes und eines Kupfersalzes umsetzt und das Reaktionsprodukt im basischen Medium verseift und decarboxyliert.

2. Verfahren nach Anspruch 1, dadurch gekennzeichnet, daß man Brom- oder Iod-bis-(trifluormethyl)-benzole der Formel (I) einsetzt 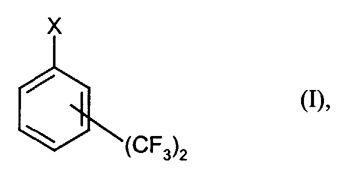
in der X für Brom oder Iod steht,
und [Bis-(trifluormethyl)-phenyl]-essigsäuren der Formel (II) herstellt 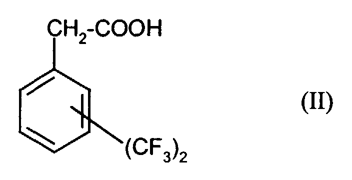 und als Malonsäure-di-C₁-C₄-alkylester solcher der Formel (III) verwendet
ROOC-CH₂-COOR (III),
in der die beiden Reste R gleich oder verschieden sind und jeweils für C₁-C₄-Alkyl stehen.

3. Verfahren nach Anspruch 2, dadurch gekenzeichnet, daß in den Formeln (I) und (II) die beiden Trifluormethylgruppen sich in 2,4- oder 3,5-Stellung zum Bromatom bzw. zur CH₂-COOH-Gruppe befinden.

4. Verfahren nach Ansprüchen 1 bis 3, dadurch gekennzeichnet, daß man als Deprotonierungsagenz Erdalki- und/oder Alkalimetallhydride, -amide, -alkoholate, -carbonate und/oder -hydrogencarbonate, Natrium-, Kalium- und/oder Ammoniumcarbonat, Natrium- und/oder Kaliumhydrogencarbonat und/oder tertiäre Amine einsetzt.

5. Verfahren nach Ansprüchen 1 bis 4, dadurch gekennzeichnet, daß man als Deprotonierungsagenz Alkoholate in fester Form oder gelöst in einem Alkohol einsetzt.

6. Verfahren nach Ansprüchen 1 bis 5, dadurch gekennzeichnet, daß man als Kupfersalze Kupfer(I)-salze einsetzt.

7. Abänderung des Verfahrens nach Ansprüchen 1 bis 6, dadurch gekennzeichnet, daß man zur Herstellung von [Bis-(trifluormethyl)-phenyl]-essigsäureestern das nach der Umsetzung mit einem Deprotonierungsagenz und Kupfersalz vorliegende Reaktionsgemisch abkühlt, mit Säure einen pH-Wert im Bereich von 5 bis 9 einstellt, die festen Bestandteile des Reaktionsgemisches abtrennt, den Rückstand mit einem Ether oder einem Alkohol wäscht und aus dem mit der Waschflüssigkeit vereinten Filtrat das Produkt durch Destillation unter vermindertem Druck gewinnt.

8. Verfahren nach Ansprüchen 1 bis 7, dadurch gekennzeichnet, daß man es in Gegenwart von aromatischen Kohlenwasserstoffen, halogierten aromatischen Kohlenwasserstoffen, Ethern, Amiden, N-Oxiden, Estern, Sulfonen und/oder Alkoholen durchführt.

9. Verfahren zur Herstellung eines Gemisches der Ester der Formeln (IV) und (V) 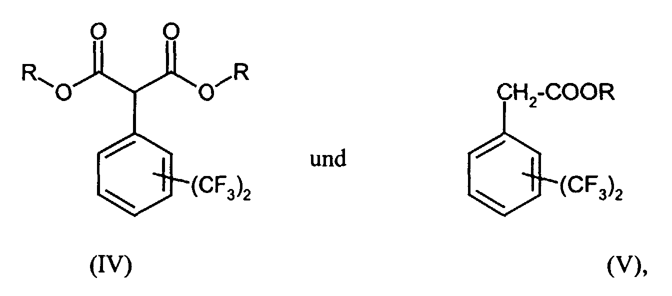 in denen die Reste R gleich oder verschieden sind und jeweils für C₁-C₄-Alkyl stehen, dadurch gekennzeichnet, daß man zunächst entsprechend Anspruch 1 verfährt, dann vor der Verseifung und vollständigem Decarboxylieren das vorliegende Reaktionsgemisch mit Wasser und Säure versetzt und erwärmt und das dann vorliegende Gemisch durch Extraktion und Destillation aufarbeitet.

10. Verfahren zur Herstellung von Phenylmalonsäuredialkylestern der Formel (IV) 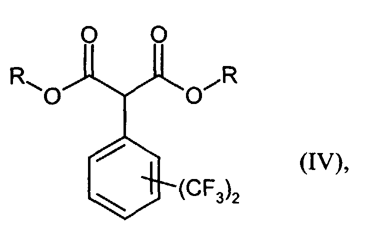 in der die beiden Reste R gleich oder verschieden sind und jeweils für C₁-C₄-Alkyl stehen,
dadurch gekennzeichnet, daß man entsprechend Anspruch 8 arbeitet und anschließend mittels Säulenchromatographie, fraktionierter Destillation oder Dünnschichtdestillation aufarbeitet.

11. Verfahren zur Herstellung von Phenylessigsäurealkylestern der Formel (V) 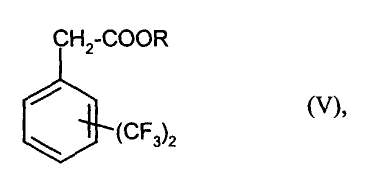 in der
R für C₁-C₄-Alkyl steht,
dadurch gekennzeichnet, daß man entsprechend Anspruch 7 arbeitet und anschließend durch Destillation bei vermindertem Druck aufarbeitet.

12. [Bis-(trifluormethyl)-phenyl]-malonsäuredialkylester der Formel 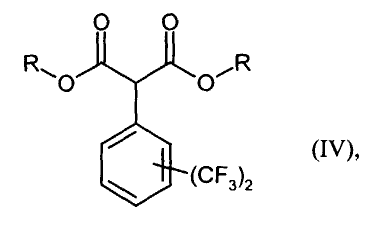 in der die beiden Reste R gleich oder verschieden sind und jeweils für C₁-C₄-Alkyl stehen.
